Europäisches Patentamt

(19) European Patent Office

Office européen des brevets

(11) Publication number : **0 361 385 B1**

(12) EUROPEAN PATENT SPECIFICATION

(45) Date of publication of patent specification :
07.01.93 Bulletin 93/01

(51) Int. Cl.⁵ : **A61L 9/015**, B01J 23/00

(21) Application number : 89117739.6

(22) Date of filing : 26.09.89

(54) **Deodorizing method and deodorizing catalyst.**

(30) Priority : 26.09.88 JP 240549/88
28.11.88 JP 301404/88
28.12.88 JP 334349/88

(43) Date of publication of application :
04.04.90 Bulletin 90/14

(45) Publication of the grant of the patent :
07.01.93 Bulletin 93/01

(84) Designated Contracting States :
DE FR GB IT NL

(56) References cited :
EP-A- 0 257 307
PATENT ABSTRACTS OF JAPAN, vol. 13, no.
249 (C-605)[3597], 9th June 1989; & JP-A-1 56
124 (NIPPON SHOKUBAI KAGAKU
KOGYOCO., LTD) 03-03-1989
PATENT ABSTRACTS OF JAPAN, vol. 13, no.
79 (C-571)[3427], 22nd February 1989; & JP-
A-63 267 362 (NIPPON SHOKUBAI
KAGAKUKOGYO CO., LTD) 04-11-1988

(56) References cited :
WORLD PATENTS INDEX (LATEST), acces-
sion no. 82-75385E, week 36, Derwent Publi-
cations Ltd, London, GB; & JP-A-57 122
924(TOKYO KOKI K.K.) 31-07-1982
PATENT ABSTRACTS OF JAPAN, vol. 10, no.
269 (C-372)[2325], 12th September 1986; &
JP-A-61 93 835 (MITSUO MATSUI)12-05-1986

(73) Proprietor : SAKAI CHEMICAL INDUSTRY CO.,
LTD.,
1-23, Ebisunochonishi 1-cho
Sakai-shi, Osaka 590 (JP)

(72) Inventor : Yoshimoto, Masafumi
2-19, Yachiyodori
Sakai-shi Osaka, 590 (JP)
Inventor : Nakatsuji, Tadao
10-13, Mamigaoka 1-chome Kashiba-cho
Kitakatsuragi-gun Nara, 639-02 (JP)
Inventor : Nagano, Kazuhiko
Yasuda Manshon 411 5-170, Bodai-cho
Sakai-shi Osaka, 591 (JP)

(74) Representative : Hansen, Bernd, Dr.rer.nat. et
al
Hoffmann, Eitle & Partner Patentanwälte
Arabellastrasse 4 Postfach 81 04 20
W-8000 München 81 (DE)

## Description

### BACKGROUND OF THE INVENTION

The present invention relates to a deodorizing method for destroying compounds of emitting an offensive smell, contained in gas or the like (hereinafter referred to as stinking components) and deodorizing catalyst for using of this present method.

Examples of a conventional method of destroying the stinking components contained in gas, include (i) an adsorptive deodorizing method using a porous substance such as activated carbon, zeolite or the like, (ii) a wet-treatment deodorizing method using an oxidizing agent or a reducing agent, (iii) an ozonolysis deodorizing method and (iv) the like.

However, any of the conventional deodorizing method above mentioned (hereinafter referred to as conventional methods) does not fully achieve a satisfactory deodorizing.

More specifically, in the adsorptive deodorizing method, the adsorbent needs to be regenerated or the like since its adsorbing ability expires in a limited period of time. This presents the problem that the deodorizing apparatus requires much labor and expense for maintenance.

The wet treatment deodorizing method involves a troublesome treatment of additives such as an oxidizing agent or the like.

Finally, the ozonolysis deodorizing method includes no such problems as above mentioned, but requires decomposition of ozone contained in gas submitted to deodorizing in view of insufficient removal of the stinking components by oxidative destruction and prevention of environmental pollution which may provoke a respiratory disease or the like.

The solid type catalyst of which whole is shaped by catalytic active ingredient, e.g. manganese, titanium and clay in the form of honeycomb, was used as an oxidative destruction catalyst to remove the stinking components in the ozonolysis deodorizing method. According to the conventional solid catalyst, however since the saturated absorption amount of stinking components in the catalyst is large, when the condition change is occurred in a refrigeration or the like, for example the reaction temperature is risen, concentration of stinking components becomes low, the stinking components are released from the catalyst to the air, and as a result, concentration of stinking components in the air is increased.

### SUMMARY OF THE INVENTION

Main object of the present invention is to provide an ozonolysis deodorizing method which is superior to the conventional method in ability of decomposing and removing stinking components and by which unreacted ozone hardly remains after deodorizing treatment.

Another object of this invention is to provide a deodorizing catalyst for using in the ozonolysis deodorizing method, by which stable rate of decomposing stinking compounds can be obtained, even if changing the reaction condition e.g. rising the reaction temperature, lowering the concentration of stinking component or the like.

To achive the object above mentioned, the deodorizing method in accordance with the present invention (hereinafter referred to as the present method) is adapted to remove stinking components by oxidative destruction with ozone in the presence of a catalyst comprising (i) at least one of metal oxide, each metal of which is selected from the group consisting of copper, manganese, cobalt, iron and nickel, and (ii) at least one selected from the group consisting of titanium dioxide, silver oxide and gold, or comprising manganese dioxide and clay.

The deodorizing catalyst for using in this present invention comprises an inactive carrier, and active components carried on said inactive carrier at the range from 10 to 200 μm of thickness.

### BRIEF DESCRIPTION OF THE DRAWINGS

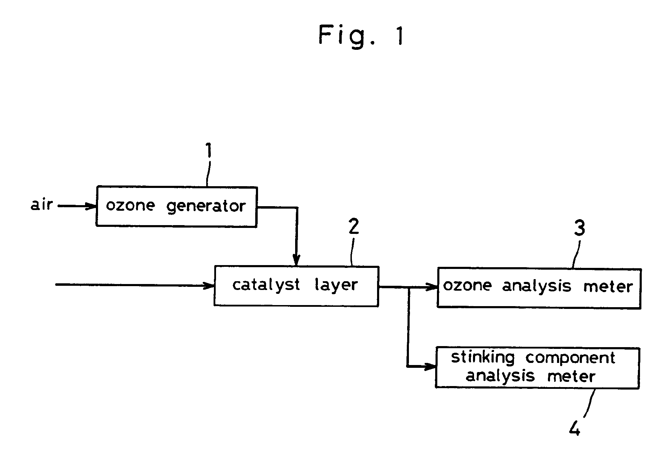

Figure 1 is a flowsheet of a catalyst activity test emplyed in Examples.

### DETAILED DESCRIPTION OF THE INVENTION

Examples of the stinking components to be removed by the present method include ammonia, trimethyl amine, hydrogen sulfide, methyl mercaptan, methyl sulfide, methyl disulfide, acetaldehyde, styrene, methyl ethyl ketone, acrolein, propionaldehyde, butyl alchol, phenol, cresol, diphenyl ether, acetic acid, propionic acid, n-pentanoic acid, metylamine, dimethylamine, skatole, dimetyltioether, dimetyl mercaptan, hydrogen chloride and allyl chloride.

2

The present method may be used for deodorizing an exhaust gas dischanged, for example, from sewage disposal plants, refuse disposal plants, printing factories, plating factories, general chemical factories and the like.

The catalyst comprising (i) at least one of metal oxide, each metal of which is selected from the group consisting of copper, manganese, cobalt, iron and nickel, and (ii) at least one of selected from the group consisting of titanium dioxide and silver oxide, and gold, include binary-catalyst such as $MnO_2$-$TiO_2$, $CuO$-$TiO_2$, $Co_3O_4$-$TiO_2$, $Fe_2O_3$-$TiO_2$ and $Fe_2O_3$-$Au$, and ternary catalyst such as $MnO_2$-$Co_3O_4$-$TiO_2$, $CuO$-$MnO_2$-$Ag_2O$, $MnO_2$-$Co_3O_4$-$Ag_2O$, $NiO$-$MnO_2$-$TiO_2$ or the like.

A preferable concentration (% by weight) of at least one of metal oxide, each metal of which is selected from the group consisting of Cu, Mn, Co, Fe and Ni, is in a range from 25 to 95 % as converted into the concentration of the metal alone. When the concentration of at least one of metal oxide is less than 25% as converted into the concentration of the metal alone, the catalyst cannot remove the stinking components sufficiently. When the concentration of at least one of metal oxide is more than 95% as converted into the concentration of the metal alone, the catalyst does not have enough durability.

A preferable concentration (% by weight) of at least one of metal oxide, each metal of which is selected from the group consisting of Ti and Ag, and/or Au, is in a range from 5 to 75% as converted into the concentration of the metal(s) alone. When the concentration of at least one of metal oxide is less than 5% as converted into the concentration of the metal alone, the catalyst does not have enough durability. When the concentration of at least one of metal oxide is more than 75% as converted into the concentration of the metal alone, the catalyst cannot remove the stinking components sufficiently.

In the catalyst comprising of manganese dioxide and clay, or manganese dioxide, clay and titanium dioxide, a preferably concentration (% by weight) of the manganese dioxide is in the range from 20 to 90 %. When concentration of the manganese dioxide is less then 20 %, the catalyst does not have enough active component. When concentration of the manganese dioxide is more then 90 %, capacity of the catalyst is not improved in proportion to addition of manganese dioxide.

Also, the capacity of above mentioned catalyst is improved by substituting a part of manganese to at least one of metal oxide, each metal of which is selected from the group consisting of Cu, Co, Fe, Ni and Ag. A preferably rate of the substitution as converted into oxide, is in a range from 1 to 30 %. When the rate of substitution is less than 1 %, the capacity of the catalyst is not improved. When the substitution rate is more than 30 %, capacity of the catalyst is not improved in proportion to the substitution.

The substituted catalyst mentioned above, includes $MnO_2$-$CuO$-clay, $MnO_2$-$Co_3O_4$-clay, $MnO_2$-$Fe_2O_3$-clay, $MnO_2$-$NiO$-clay, $MnO_2$-$Ag_2O$-clay, $MnO_2$-$CuO$-clay-$TiO_2$, $MnO_2$-$Co_3O_4$-clay-$TiO_2$, $MnO_2$-$Fe_2O_3$-clay-$TiO_2$, $MnO_2$-$NiO$-clay-$TiO_2$, $MnO_2$-$Ag_2O$-clay-$TiO_2$ and the like. The above mentioned clay is the bedded clay mineral which is mainly composed of biroferait, talc, mica, chlorite, montmorillonite, caolin, hairosaite and the like. Example of the clay is Kibushi clay, Gairome clay or the like.

The catalyst for using in the present method is not particularly limited in shape, but may be in any form such as honeycomb, pellet, cylinder, plate, pipe or the like.

The concentration (% by weight) of an active componets contained in the catalyst is preferably 50% or more, and more preferably 75 % or more. When the concentration of the active componets in the catalyst is less than 50 %, the catalyst cannot remove the stinking components sufficiently.

The catalyst may be manufactured by a known method suitably selected out of impregnation, kneading, coprecipitation, precipitation, oxide mixing and the like. In production of the catalyst, there may be added (i) a forming assistant such that the catalyst easily gets into shape, or (ii) a reinforcing agent such as inorganic fiber or an organic binder to improve the mechanical strength or the like.

Also, the catalyst of this invention can be produced by supporting active components on the carrier. The carrier is not particularly limited in shape, but may be in any form such as honeycomb, pellet, cylinder, plate, pipe or the like. The carrier is not particularly limited in material, but may be made of urethane foam, clay or the like.

The preferable thickness of the active components on the carrier is in the range from 10 to 200 $\mu$m. When the thickness of that is in the range, saturated absorption amount of stinking components in the catalyst is adjusted appropriately. Accordingly, the catalyst presents stable removing rate of stinking component, even if changing the reaction condition, e.g. rising the reaction temperature, lowering the concentration of stinking components or the like. When the thickness of that is more than 200 $\mu$m, the saturated absorption amount of stinking components in the catalyst is too much. And when the condition changed likely to above mentioned, the stinking compound absorbed in the catalyst is released from the catalyst to the air, as a result, the catalyst becomes generator of stench. When the thickness of that is less than 10 $\mu$m, the catalyst does not have enough capacity removing the stinking components.

A suitable amount of ozone ($O_3$) which is used together with the catalyst at the time of deodorizing, depends on the types and concentrations of stinking components to be removed, the reaction temperature, the type and amount of the catalyst, and the like. For example, when gas containing $H_2S$ as the stinking components is to be deodorized, it is preferable that 1 to 2 mols of $O_3$ coexists per mol of $H_2S$. For gas containing $NH_3$ as the stinking components, it is preferable that 1 to 3 mols of $O_3$ coexists per mol of NH3. For gas containing methyl mercaptan as the stinking components, it is preferable that 1 to 4 mol of $O_3$ per mol of methyl mercaptan.

If gas to be deodorized contains stinking components in high concentration, the concentration $O_3$ may exceed the preferable range above mentioned to improve the stinking component removing rate. However, if excessive, surplus $O_3$ may remain after deodorizing. Accordingly, attention should be paid such that no excessive $O_3$ coexists to prevent the generation of such residue.

The reaction temperature at time of deodorizing is preferably in a range from 0 to 40 °C and more preferably in a range from 10 to 30 °C. The temperature less than 0 °C causes the reaction speed to be slower, while the temperature exceeding 40 °C requires an additional energy for increasing the temperature, resulting in poor economy.

Preferably, the catalyst comes in contact with the reaction gas in a range of area velocity (AV) from 5 to 50. The area velocity less than 5 requires a greater amount of catalyst, while the area velocity exceeding 50 causes the decomposition efficiency to be lowered, failing to achive the desired decomposition efficiency. it is here noted that the area velocity refers to a value obtained by dividing the reaction amount ($Nm^3/u$, u:Hr) by the gas contact area per catalyst in unit volume ($m^2/m^3$).

EXAMPLES

The following description will discuss, in more detail, examples of the present invention. It is noted that the present invention should not be limited to the following examples.

Example 1

Kibusushi clay was dried for 18 hours at 100 °C, after that it was crushed by a sample mill having the 0.5 mm /$\phi$ screen. 20 kg of the results was mixed with 1 kg of methyl cellulose type binder (Yuken Industry Co., YB-32) and water. The mixture was kneaded thoroughly by a neader. After that, the mixture was put into the orga-screw type extruder, the honeycomb was obtained. In such a case, the water content was adjusted so as to have 30 - 35 $kg/cm^2$ of pressure. The obtained honeycomb was air-dried at ordinary temperature, and it was heated up to 500 °C at the rate of 5 °C by a hour. After it keep 500 °C for 3 hours, it was cooled down at the rate of 10 °C by a hour. Thus, the honeycomb type carrier having open area rate of 64 % and pitch of 4.0 mm was obtained.

704 g of $MnO_2$ having a specific surface area of 48 $m^2/g$ was added to 1034 ml of a titania sol ($TiO_2$ concentration : 150 g/$\ell$). To the mixture, 250 g of grass bead was added to adjust the degree of milling. The resultant mixture was agitated and mixed for 30 minutes. The grass bead was apart from the mixture to produce the slurry. After the slurry was diluted with 300 ml of water, the slurry was impregnated into the corrugated honeycomb. The exceeded slurry was excluded from the honeycomb, and the honeycomb was air-dried and baked. Thus, there was prepared a binary-catalyst carrying 10 $\mu$m in thickness of $MnO_2$-$TiO_2$ (weight ratio of 82 :18) with 43 % of carry rate. The open area rate of the catalyst is 63 %, the area contacted with gas at a volume (hereinafter referd to as AP) is 795 $m^2/m^3$. The thickness of the $MnO_2$-$TiO_2$ was measured by electron probe micro analyser (EPMA).

Example 2

There was prepared a binary-catalyst carrying $MnO_2$-$TiO_2$ having open area range 60% and Ap 775 $m^2/m^3$, in the same manner as that of Example 1, except that carry weight rate of the catalyst was 26.5 % and average thickness of $MnO_2$-$TiO_2$ was about 50 $\mu$m in Example 2.

Example 3

There was prepared a binary catalyst carrying $MnO_2$-$TiO_2$ having open area rate 56% and Ap 750 $m^2/m^3$, in the same manner as that of Example 1, except that $MnO_2$-$TiO_2$ sol slurry was not diluted with water, average carry weight rate of catalyst was 40.7 % and average thickness of $MnO_2$-$TiO_2$ was about 100 $\mu$m in Example 3.

Example 4

There was prepared a binary-catalyst carrying $MnO_2$-$TiO_2$ having open area rate 49% and Ap 700 $m^2/m^3$, in the same manner as that of Example 1, except that $MnO_2$-$TiO_2$ sol slurry was not diluted with water, carry weight rate of the catalyst was 65.1 % and average thickness of $MnO_2$-$TiO_2$ was about 200 $\mu$m in Example 4.

Example 5

There was prepared a binary-catalyst carrying $MnO_2$-$TiO_2$ having open area rate 64% and Ap 798 $m^2/m^3$, in the same manner as that of Example 1, except that carry weight rate of the catalyst was 2.2 % and average thickness of $MnO_2$-$TiO_2$ was about 5 $\mu$m in Example 5.

Example 6

There was prepared a binary-catalyst carrying $MnO_2$-$TiO_2$ having open area rate 46% and Ap 675 $m^2/m^3$, in the same manner as that of Example 1, except that $MnO_2$-$TiO_2$ sol slurry was not diluted with water, carry weight rate of the catalyst was 72.8 % in and average thickness of $MnO_2$-$TiO_2$ was about 250 $\mu$m Example 6.

Catalyst Activity Test

Each catalyst of Examples 1 to 6 was submitted to a catalyst activity test with the use of the test method having a flowsheet as shown in Fig. 1, under reaction conditions discussed later.

In Fig. 1, stinking components $H_2S$, $NH_3$ and methyl amine contained in gas to be deodorized are introduced into a catalyst layer 2. The stinking components thus introduced are decomposed by ozone $O_3$ introduced from an ozone generator 1 to the catalyst layer 2. A portion of the gas after decomposed and deodorized is introduced into an ozone analysis 3, in which the remaining ozone $O_3$ is subjected to quantitative analysis. The remaining potion of the gas after decomposed and deodorized is introduced to a stinking component analysis meter 4 including two gas chromatographs (designed for analyzing $H_2S$ or methyl amine) and one $NH_3$ meter. These devices are adapted to carry out quantitative analysis of stinking components.

(Reaction Conditions I)

Space velocity : 20,000/Hr
Reaction temperature : 20 °C
Concentration of Stinking components
/Concentration of ozone components :
$H_2S$/ozone : 10/20 ppm
$NH_3$/ozone: 10/30 ppm
methyl mercaptan/ozone 5/10 ppm

(Reaction Conditions II)

After catalyst Activity Test was carried out for 24 hours under Reaction Condition I, reaction temperature was risen to 50 °C. After 5 minutes, concentrations of stinking components and ozone were measured.

(Reaction Conditions III)

After catalyst Activity Test carried out for 24 hours under Reaction Condition I, concentration of stinking components was changed to the following. After 5 minutes, concentrations of stinking components and ozone were measured.
concentration of $H_2S$ : 1 ppm
concentration of $NH_3$ : 1 ppm
concentration of methyl mercaptan : 1 ppm
The test result are shown in the Table 1.

Table 1

| | Stinking components | Reaction conditions I | | Reaction conditions II | | Reaction conditions III | |
|---|---|---|---|---|---|---|---|
| | | Rate of removing stinkin components (%) | Ozon residual rate (%) | Rate of removing stinkin components (%) | Ozon residual rate (%) | Rate of removing stinkin components (%) | Ozon residual rate (%) |
| Example 1 | $H_2S$ | 91 | 0.8 | 93 | 0 | 91 | 0.6 |
| | $NH_3$ | 82 | 2.1 | 85 | 1.5 | 83 | 1.8 |
| | Methyl-mercaptan | 95 | 0 | 98 | 0 | 96 | 0 |
| Example 2 | $H_2S$ | 94 | 0.2 | 95 | 0 | 94 | 0.2 |
| | $NH_3$ | 86 | 1.8 | 88 | 0.9 | 85 | 1.9 |
| | Methyl-mercaptan | 99 | 0 | 99 | 0 | 99 | 0 |
| Example 3 | $H_2S$ | 94 | 0.1 | 98 | 0 | 93 | 0 |
| | $NH_3$ | 85 | 1.5 | 89 | 0.8 | 86 | 1.3 |
| | Methyl-mercaptan | 99 | 0 | 100 | 0 | 99 | 0 |
| Example 4 | $H_2S$ | 98 | 0.5 | 94 | 0 | 94 | 0.1 |
| | $NH_3$ | 86 | 2.3 | 83 | 1.6 | 81 | 2.2 |
| | Methyl-mercaptan | 96 | 0 | 98 | 0 | 96 | 0 |
| Example 5 | $H_2S$ | 76 | 3.5 | 79 | 3.0 | 77 | 3.1 |
| | $NH_3$ | 62 | 5.1 | 64 | 4.8 | 61 | 4.8 |
| | Methyl-mercaptan | 79 | 0.5 | 80 | 0.2 | 81 | 0.5 |
| Example 6 | $H_2S$ | 98 | 0.2 | 75 | 0 | 77 | 0 |
| | $NH_3$ | 86 | 1.3 | 68 | 0 | 65 | 0 |
| | Methyl-mercaptan | 99 | 0 | 81 | 0 | 82 | 0 |

EP 0 361 385 B1

As apparent from Table 1, any of catalyst obtained in Example 1 to 6 presents high and stable rate of removing stinking components. Especialy, the catalyst obtained in Example 1 to 4, which has 10 to 200 $\mu$m in thickness of active components on carrier, presents stable rate of removing stinking components.

Example 7

30 g of $MnO_2$ having a specific surface area of 48m$^2$/g and 70 g of anatase type $TiO_2$ having a specific surface area of 85 m$^2$/g were added to 170 ml of a titania sol ($TiO_2$ concentration : 150 g/$\ell$). The mixture was agitated and mixed for 30 minutes to produce slurry, which was impregnated into a corrugated honeycomb made of ceramic fibers having open area rate of 81% and pitch of 4.0 mm. Thus there was prepared a binary-catalyst carrying about 50 $\mu$m in average thickness of $MnO_2$-$TiO_2$ (mol ratio of 24 : 76) with a carry rate of 101%.

Example 8

There was prepared a binary-catalyst carrying about 50 um in average thickness of $CuO$-$TiO_2$ (mol ratio of 24 : 76) with a carry rate of 91%, in the same manner as that of Example 7, except that $CuO$ having a specific surface area of 62 m$^2$/g was used in Example 8, instead of 30 g of $MnO_2$ having a specific surface area of 48m$^2$/g used in Example 7.

Example 9

There was prepared a binary-catalyst carrying about 50 $\mu$m in average thickness of $Co_3O_4$-$TiO_2$ (mol ratio of 24 : 76) with a carry rate of 91%, in the same manner as that of Example 7, except that $Co_3O_4$ having a specific surface area of 53 m$^2$/g was used in Example 9, instead of 30 g of $MnO_2$ having a specific surface area of 48m$^2$/g used in Example 7.

Example 10

There was prepared a binary-catalyst carrying about 50 $\mu$m in average thickness of $Fe_2O_3$-$TiO_2$ (mol ratio of 24 : 76) with a carry rate of 78 %, in the same manner as that of Example 7, except that $Fe_2O_3$ having a specific surface area of 53 m$^2$/g was used in Example 10, instead of 30 g of $MnO_2$ having a specific surface area of 48m$^2$/g used in Example 7.

Example 11

There was prepared 500 ml of a water solution containing 112 g of manganese acetate (4 hydrates), 182 g of cobalt nitrate (6 hydrates) and 63 g of metatitanic acid ($TiO_2$ concentration of 40%). while the solution was agitated, ammonia water was gradually added to the solution, causing the same to be neutrized. Thus, a slurry-like precipitate was produced with the final pH of 7.0. The slurry was impregnated into the same corrugated honeycomb as in Example 7 was then impregnated with this precipitate. The honeycomb was calcined for three hours at 450 °C. Thus, there was prepared a ternary-catalyst carrying about 50 um in average thickless of $MnO_2$-$Co_3O_4$-$TiO_2$ (mol ratio of 25 : 50 : 25) with a carry rate of 89 % and having a specific surface area of 72 m$^2$/g.

Example 12

There was prepared 500 ml of a water solution containing 17.8 g of manganese acetate (4 hydrates), 288 g of cobalt nitrate (6 hydrates) and 1.5 g of silver nitrate. While the solution was agitated, a water solution of ammonium carbonate was added to the first mentioned solution, causing the same to be neutrized, Thus, a slurry-like precipitate was produced with the final pH of 7.0. The slurry was impregnated into the same corrugated honeycomb as in Example 7. The honeycomb was then calcined for three hours at 450 °C. Thus, there was prepared a ternary-catalyst carrying about 50 $\mu$m in average thickness of $Co_3O_4$-$MnO_2$-$TiO_2$ (mol ratio of 20 : 40 : 1) with a carry rate of 92% and having a specific surface area of 65 m$^2$/g.

Example 13

There was prepared 500 ml of a water solution containing 74.4 g of cupric nitrate (6 hydrates), 17.8 g of manganese acetate (4 hydrates) and 1.5 g of silver nitrate. While the solution was agitated, a water solution of ammonium cabonate was added to the first mentioned solution, causing the same to be nutrized, Thus, a slurry like precipitate was produced with the final pH of 7.0. This slurry was impregnated into the same corrugated honeycomb as in Example 7. The honeycomb was then calcined for three hours at 450 °C. Then, there was prepared a ternary-catalyst carrying about 50 $\mu$m in average thickness of $CuO-MnO_2-Ag_2O$ (mol ratio of 20 : 40 : 1) with a carry rate of 87% and having a specific surface area of 71 $m^2$/g.

## Example 14

There was prepared 500 ml of a water solution containing 112 g of manganese acetate (4 hydrates), 195 g of nickel nitrate (6 hydrates) and 63 g of metatitanic acid ($TiO_2$ concentration of 40%). while the solution was agitated, ammonia water was gradually added to the solution. Thus, a slurry-like precipitate was produced with the final pH of 7.0. The slurry was impregnated into the same corrugated honeycomb as in Example 7. The honeycomb was was calcined for three hours at 450 °C, Thus, there was prepared a ternary-catalyst carrying about 50 um in average thickless of $MnO_2-Co_3O_4-TiO_2$ (mol ratio of 25 : 50 : 25) with a carry rate of 94 % and having a specific surface area of 80 $m^2$/g.

## Example 15

There was prepared ternary-catalysis carrying about 50 um in average thickness of $NiO-MnO_2-TiO_2$ (mol ratio of 20 : 40 : 1) with specific surface area of 77 $m^2$/g, a carry rate of 98 %, in the same manner of Example 12, except that 309 g of Nickel nitrate (4 hydrates) used in Example 15, instead of 288 g of cobalt nitrate (6 hydrates) in Example 12.

## Example 16

There was prepared binary-catalysis carrying about 50 um in average thickness $Fe_2O_3-Ag_2O$ (mol ratio of 95 : 5) with a carry rate of 104 % , in the same manner of Example 7, except that Au colloid which was produced by 30 % hydrogen peroxide added to chloroauric acid at pH 8.0 used in Example 16, instead of 70 g of anatase type $TiO_2$ and 170 ml of a titania solution used in Example 12.

## Comparative Example 1

30 g of $MnO_2$ having a specific surface area of 48 $m^2$/g and 70 g of a mixture ($MiO_2$ : $SiO_2$ = 1 : 1) of titanium tetrachloride with a silica sol were agitated and mixed. While agitating and mixing, ammonia gas was blown such that the resultant mixture was neutrized, thereby to produce a slurry like precipitate. After sufficiently washed with water, the precipitate was then calcined for three hours at 500 °C. Thus, there was prepared a ternary catalyst $MnO_2-TiO_2-SiO_2$ (mol ratio of 3 : 3.5 : 3.5) having a specific surface area of 162 $m^2$/g.

## Comparative Examples 2

100 g of $TiO_2$ having a specific surface area of 85 $m^2$/g was calcined for 3 hours at 500 °C. Thus, a catalyst which is consisted of $TiO_2$ was obtained.

## Comparative Example 3

100 g of $MnO_2$ having a specific surface area of 85 $m^2$/g was baked for 3 hours at 500 °C. Thus, a catalyst which is consisted of $MnO_2$ was obtained.

## Catalyst Activity Test

Each catalyst of Examples 7 to 16 and Comparative Examples 1 to 3 was submitted to a catalyst activity test with the use of the test method having a flowsheet as shown in Fig. 1, under reaction conditions discussed later.

(Reaction Conditions)

   Space velocity : 20,000/Hr
   Reaction temperature : 20 °C

Stinking components : $H_2S$, $NH_3$ or methyl mercaptan
The test result are shown in the Table 2.

Table 2

| | Stinking components | Concentration of stinking (ppm) | Concentration of ozon (ppm) | Rate of removing stinkin components (%) | Ozon residual rate (%) |
|---|---|---|---|---|---|
| Example 7 | $H_2S$ | 10 | 20 | 98 | 0 |
| Example 8 | $H_2S$ | 10 | 20 | 91 | 2 |
| Example 9 | $H_2S$ | 10 | 20 | 86 | 3.2 |
| Example 10 | $H_2S$ | 10 | 20 | 98 | 0 |
| Example 11 | $H_2S$ | 10 | 20 | 99 | 0 |
| | $NH_3$ | 10 | 30 | 90 | 0.4 |
| | Methyl-mercaptan | 5 | 10 | 100 | 0 |
| Example 12 | $NH_3$ | 10 | 30 | 95 | 0 |
| Example 13 | $NH_3$ | 10 | 30 | 92 | 1.1 |
| Example 14 | $NH_3$ | 10 | 30 | 84 | 1.5 |
| Example 15 | $NH_3$ | 10 | 30 | 81 | 1.8 |
| Example 16 | $NH_3$ | 10 | 30 | 90 | 0.2 |
| Comparative Example 1 | $H_2S$ | 10 | 20 | 61 | 4.0 |
| | $NH_3$ | 10 | 30 | 73 | 1.8 |
| | Methyl-mercaptan | 5 | 10 | 88 | 1.0 |
| Comparative Example 2 | $H_2S$ | 10 | 20 | 12 | 72 |
| | $NH_3$ | 10 | 30 | 8 | 89 |
| | Methyl-mercaptan | 5 | 10 | 36 | 55 |
| Comparative Example 3 | $H_2S$ | 10 | 20 | 29 | 0 |
| | $NH_3$ | 10 | 30 | 13 | 0 |
| | Methyl-mercaptan | 5 | 10 | 49 | 0 |

As apparent from the Table 2, any of the catalysts obtained in Examples 7 to 16 presents a higher stinking component removing rate than the catalysts obtained in Comparative Example 1 to 3.

Except for the catalyst of Example 7, any of the catalysts obtained in Example 7 to 16 presents a lower ozone residual rate than the catalysts obtained in Comparative Example 1 to 3.

## Example 17

704 g of $MnO_2$ having a specific surface area of 48 m²/g and 155 g Kibushi clay were mixed with 1 $\ell$ of water. To the mixture, 250 g of grass bead was added to adjust the degree of milling, and was agitated and mixed for 30 minutes to produce slurry. The slurry was impregnated into a corrugated honeycomb made of ceramic fibers having open area rate of 81% and pitch of 4.0 mm. Thus, there was prepared a binary-catalyst carrying about 50 µm in average thickness of $MnO_2$-clay (weight rate of 82 : 18) with a carry rate 95%.

## Example 18

There was prepared a ternary-catalyst carrying about 50 µm in average thickness of $MnO_2$-$TiO_2$-clay (weight rate of 82 : 9 : 9) with carry rate of 95 %, in the same manner as that Example 17, except that 78 g of Kibushi clay and 517 ml of titania sol ($TiO_2$ concentration : 150 g/$\ell$) was mixed with 500 ml of water in Example 18, instead of 704 g of $MnO_2$ and 155 g Kibushi clay mixed with 1 $\ell$ of water in Example 17.

## Example 19

There was prepared a binary-catalyst carrying 50 µm in average thickness of $MnO_2$-clay (weight ratio of 24 : 76) with carry rate of 103 %, in the same manner as that Example 17, except that 95 g of Kibushi clay and 30g of $MnO_2$ was mixed with 500 ml of water in Example 20, instead of 704 g of $MnO_2$ and 155 g Kibushi clay mixed with 1 $\ell$ of water in Example 17.

## Example 20

There was prepared a ternary-catalyst carrying 50 µm in average thickness of $MnO_2$-$TiO_2$-clay (weight ratio of 24 : 20 : 56) with carry rate of 101 %, in the same manner as that Example 19, except that 70 g of Kibushi clay, 30g of $MnO_2$ and 170 ml of titania sol was mixed with 500 ml of water in Example 20, instead of 95 g of Kibushi clay and 30g of $MnO_2$ mixed with 500 ml of water in Example 19.

## Example 21

There was prepared a ternary-catalyst carrying 50 µm in average thickness of $MnO_2$-CuO-clay (weight ratio of 77 : 5 : 18) with carry rate of 97 %, in the same manner as that Example 17, except that 43 g of CuO and 661 g of $MnO_2$ used in Example 21, instead of 704 g of $MnO_2$ used in Example 17.

## Example 22

There was prepared a ternary-catalyst carrying 50 µm in average thickness of $MnO_2$-$Co_3O_4$-clay (weight ratio of 80 : 2 : 18) with carry rate of 101 %, in the same manner as that Example 17, except that 17 g of $Co_3O_4$ and 687 g of $MnO_2$ used in Example 22, instead of 704 g of $MnO_2$ used in Example 17.

## Example 23

There was prepared a ternary-catalyst carrying 50 µm in average thickness of $MnO_2$-$Fe_2O_3$-clay (weight ratio of 74 : 8 : 18) with carry rate of 98 %, in the same manner as that Example 17, except that 70 g of $Fe_2O_3$ and 634 g of $MnO_2$ used in Example 22, instead of 704 g of $MnO_2$ used in Example 17.

## Example 24

There was prepared a ternary-catalyst carrying 50 µm in average thickness of $MnO_2$-NiO-clay (weight ratio of 76 : 6 : 18) with carry rate of 100 %, in the same manner as that Example 17, except that 50 g of NiO and 654 g of $MnO_2$ used in Example 24, instead of 704 g of $MnO_2$ used in Example 17.

## Example 25

There was prepared a ternary-catalyst carrying 50 µm in average thickness of $MnO_2$-$Ag_2O$-clay (weight ratio of 80 : 2 : 18) with carry rate of 100 %, in the same manner as that Example 17, except that 17 g of $Ag_2O$ and 687 g of $MnO_2$ used in Example 25, instead of 704 g of $MnO_2$ used in Example 17.

Example 26

There was prepared a quartery-catalyst carrying 50 $\mu$m in average thickness of $MnO_2$-$CuO$-$TiO_2$-clay (weight ratio of 77 : 5 : 9 : 9) with carry rate of 103 %, in the same manner as that Example 21, except that 78 g of Gairome clay and 517 g of titania sol used in Example 26, instead of 155 g of Kibushi clay used in Example 17.

Example 27

There was prepared a quartary-catalyst carrying 50 $\mu$m in average thickness of $MnO_2$-$Ag_2O$-$TiO_2$-clay (weight ratio of 80 : 2 : 9 : 9) with carry rate of 103 %, in the same manner as that Example 17, except that 78 g of Gairome clay and 517 g of titania sol used in Example 27, instead of 155 g of Kibushi clay in Example 17.

Comparative Example 4

100 g of Kibushi clay having a specific surface area of 56 $m^2$/g was calcined for 3 hours at 500 °C. Thus, a catalyst consisted in Kibushi clay, was obtained.

Catalyst Activity Test

Each catalyst of Examples 17 to 27 and Comparative Examples 4 were submitted to a catalyst activity test with the use of the test method having a flowsheet as shown in Fig. 1, under the same reaction conditions as the Example 7 to 16.

The test result are shown in the Table 3.

EP 0 361 385 B1

Table 3

| | Catalyst | Stinking components | Concentration of stinking (ppm) | Concentration of ozon (ppm) | Rate of removing stinkin components (%) | Ozon residual rate (%) |
|---|---|---|---|---|---|---|
| Example 17 | $MnO_2$/clay = 82/18 | $H_2S$<br>$NH_3$<br>Methyl-mercaptan | 10<br>10<br>5 | 20<br>30<br>10 | 88<br>82<br>98 | 1.1<br>1.4<br>0.5 |
| Example 18 | $MnO_2$/$TiO_2$ /clay = 82/9/9 | $H_2S$<br>$NH_3$<br>Methyl-mercaptan | 10<br>10<br>5 | 20<br>30<br>10 | 90<br>87<br>99 | 0.8<br>1.3<br>0.3 |
| Example 19 | $MnO_2$/$TiO_2$ = 24/76 | $H_2S$<br>$NH_3$<br>Methyl-mercaptan | 10<br>10<br>5 | 20<br>30<br>10 | 83<br>83<br>95 | 3.5<br>1.6<br>0.5 |
| Example 20 | $MnO_2$/$TiO_2$ /clay = 24/20/56 | $H_2S$<br>$NH_3$<br>Methyl-mercaptan | 10<br>10<br>5 | 20<br>30<br>10 | 85<br>85<br>99 | 1.8<br>1.5<br>0.4 |
| Example 21 | $MnO_2$/CuO /clay = 77/5/18 | $H_2S$<br>$NH_3$<br>Methyl-mercaptan | 10<br>10<br>5 | 20<br>30<br>10 | 97<br>90<br>98 | 0<br>0.4<br>0 |
| Example 22 | $MnO_2$/$Co_3O_4$ /clay = 80/2/18 | $H_2S$<br>$NH_3$<br>Methyl-mercaptan | 10<br>10<br>5 | 20<br>30<br>10 | 98<br>92<br>100 | 0<br>0.2<br>0 |
| Example 23 | $MnO_2$/$Fe_2O_3$ /clay = 74/8/18 | $H_2S$<br>$NH_3$<br>Methyl-mercaptan | 10<br>10<br>5 | 20<br>30<br>10 | 99<br>89<br>100 | 0<br>0<br>0.8 |

12

Table 3 (continued)

| | Catalyst | Stinking components | Concentration of stinking (ppm) | Concentration of ozon (ppm) | Rate of removing stinkin components (%) | Ozon residual rate (%) |
|---|---|---|---|---|---|---|
| Example 24 | Mno$_2$/NiO /clay = 76/6/18 | H$_2$S NH$_3$ Methyl-mercaptan | 10 10 5 | 20 30 10 | 97 91 100 | 0 0.5 0 |
| Example 25 | MnO$_2$/Ag$_2$O /clay = 82/2/18 | H$_2$S NH$_3$ Methyl-mercaptan | 10 10 5 | 20 30 10 | 100 93 100 | 0 0.5 0 |
| Example 26 | MnO$_2$/CuO /TiO$_2$/clay =77/5/9/9 | H$_2$S NH$_3$ Methyl-mercaptan | 10 10 5 | 20 30 10 | 99 93 100 | 0 0.2 0 |
| Example 27 | MnO$_2$/Ag$_2$O /TiO$_2$/clay =80/2/9/9 | H$_2$S NH$_3$ Methyl-mercaptan | 10 10 5 | 20 30 10 | 100 94 100 | 0 0.3 0 |
| Comparative Example 4 | clay | H$_2$S NH$_3$ Methyl-mercaptan | 10 10 5 | 20 30 10 | 21 9 40 | 57 82 30 |

EP 0 361 385 B1

EP 0 361 385 B1

As apparent from Table 3, any of the catalysts obtained in Example 17 to 27 presents a higher removing rate of stinking component than that of the catalysts obtained in Comparative Example 1 to 4.

And any of the catalysts obtained in Example 17 to 27 presents a lower ozone residual rate than that of the catalyst obtained in Comparative Example 1 to 4.

These results of the Catalyst Activity Test present that the method of the present invention is effective to remove stinking components in high rate, and unreacted ozone hardly remains after deodorizing treatment.

## Claims

1. A deodorizing method for removing stinking components by oxidative destruction with ozone in the presence of a catalyst comprising, as active components;
   at least one of metal oxide, each metal of which is selected from the group consisting of copper, manganese, cobalt, iron and nickel;
   and
   at least one selected from the group consisting of titanium dioxide, silver oxide and gold.

2. A deodorizing method according to Claim 1, wherein the catalyst is selected from the group consisting of $MnO_2$-$TiO_2$, $CuO$-$TiO_2$, $Co_3O_4$-$TiO_2$, $Fe_2O_3$-$TiO_2$, $Fe_2O_3$-$Au$, $MnO_2$-$Co_3O_4$-$TiO_2$, $CuO$-$MnO_2$-$Ag_2O$, $MnO_2$-$Co_3O_4$-$Ag_2O$ and $NiO$-$MnO_2$-$TiO_2$.

3. A deodorizing method according to Claim 1, wherein the concentration of said metal oxide, each metal of which is selected from the group consisting of Cu, Mn, Co, Fe and Ni, in said active components is in a range from 25 to 95% by weight as converted into the concentration of the metal alone.

4. A deodorizing method for removing stinking components by oxidative destruction with ozone in the presence of a catalyst comprising manganese dioxide and clay as active components.

5. A deodorizing method according to Claim 4, wherein the catalyst includes titanium dioxide in addition to manganese dioxide and clay.

6. A deodorizing method according to Claim 4, wherein the the catalyst includes at least one of metal oxide, each metal of which selected from the group consisting of copper, manganese, cobalt, nickel and silver in addition to manganese dioxide and clay.

7. The use of a catalyst for removing stinking components by oxidative destruction with ozone, the catalyst being formed of an inactive carrier supporting a layer of active components having a thickness of 10-200 μm, the active components being:
   (i) at least one metal oxide in which the metal is selected from copper, manganese, cobalt, iron and nickel; and
   (ii) at least one of titanium dioxide, silver oxide and gold.

## Patentansprüche

1. Desodorierverfahren zur Entfernung von stinkenden Verbindungen durch oxidative Zerstörung mit Ozon in Anwesenheit eines Katalysators, welcher als aktive Verbindungen umfaßt:
   mindestens ein Metalloxid, dessen Metall aus der Gruppe, bestehend aus Kupfer, Mangan, Kobalt, Eisen und Nickel ausgewählt ist;
   und
   mindestens eine Verbindung, ausgewählt aus der Gruppe, bestehend aus Titandioxid, Silberoxid und Gold.

2. Desodorierverfahren gemäß Anspruch 1, worin der Katalysator aus der Gruppe ausgewählt ist, bestehend aus $MnO_2$-$TiO_2$, $CuO$-$TiO_2$, $Co_3O_4$-$TiO_2$, $Fe_2O_3$-$TiO_2$, $Fe_2O_3$-$Au$, $MnO_2$-$Co_3O_4$-$TiO_2$, $CuO$-$MnO_2$-$Ag_2O$, $MnO_2$-$Co_3O_4$-$Ag_2O$ und $NiO$-$MnO_2$-$TiO_2$.

3. Desodorierverfahren gemäß Anspruch 1, worin die Konzentration des Metalloxids, dessen Metall aus der Gruppe, bestehend aus Cu, Mn, Co, Fe und Ni, ausgewählt ist, in den aktiven Verbindungen in einem Be-

14

reich von 25 bis 95 Gew.-% vorliegt, umgerechnet in die Konzentration des Metalls allein.

4. Desodorierverfahren zur Entfernung von stinkenden Verbindungen durch oxidative Zerstörung mit Ozon in Anwesenheit eines Katalysators, welcher Mangandioxid und Ton als aktive Komponenten umfaßt.

5. Desodorierverfahren gemäß Anspruch 4, worin der Katalysator Titandioxid zusätzlich zu Mangandioxid und Ton einschließt.

6. Desodorierverfahren gemäß Anspruch 4, worin der Katalysator mindestens ein Metalloxid einschließt, dessen Metall ausgewählt ist aus der Gruppe, bestehend aus Kupfer, Mangan, Kobalt, Nickel und Silber zusätzlich zu Mangandioxid und Ton.

7. Verwendung eines Katalysators zur Entfernung von stinkenden Verbindungen durch oxidative Zerstörung mit Ozon, wobei der Katalysator aus einem inaktiven Träger gebildet ist, der eine Schicht von aktivem Komponentem mit einer Dicke von 10 bis 200 µm trägt, wobei die aktiven Komponenten sind:
(i) mindestens ein Metalloxid, worin das Metall ausgewählt ist aus Kupfer, Mangan, Kobalt; Eisen und Nickel; und
(ii) mindestens eine Verbindung, ausgewählt aus Titandioxid, Silberoxid und Gold.

**Revendications**

1. Procédé de désodorisation pour éliminer les constituants malodorants par destruction oxydante avec de l'ozone en présence d'un catalyseur comprenant comme constituants actifs :
- au moins un oxyde métallique, dont le métal est choisi parmi le cuivre, le manganèse, le cobalt, le fer et le nickel; et
- au moins une substance choisie parmi le dioxyde de titane, l'oxyde d'argent et l'or.

2. Procédé de désodorisation selon la revendication 1, dans lequel le catalyseur est choisi parmi $MnO_2$-$TiO_2$, $CuO$-$TiO_2$, $Co_3O_4$-$TiO_2$, $Fe_2O_3$-$TiO_2$, $Fe_2O_3$-$Au$, $MnO_2$-$Co_3O_4$-$TiO_2$, $CuO$-$MnO_2$-$Ag_2O$, $MnO_2$-$Co_3O_4$-$Ag_2O$ et $NiO$-$MnO_2$-$TiO_2$.

3. Procédé de désodorisation selon la revendication 1, dans lequel la concentration de cet oxyde métallique, dont chacun des métaux est choisi parmi Cu, Mn, Co, Fe et Ni dans ces constituants actifs est dans l'intervalle de 25 à 95 % en poids, exprimés en concentration du métal seul.

4. Procédé de désodorisation pour éliminer les constituants malodorants par destruction oxydante avec de l'ozone en présence d'un catalyseur comprenant du dioxyde de manganèse et de l'argile comme constituants actifs.

5. Procédé de désodorisation selon la revendication 4, dans lequel le catalyseur contient du dioxyde de iitane en plus du dioxyde de manganèse et de l'argile.

6. Procédé de désodorisation selon la revendication 4, dans lequel le catalyseur contient au moins un oxyde métallique, chacun des métaux étant choisi parmi le cuivre, le manganèse, le cobalt, le nickel et l'argent en plus du dioxyde de manganèse et de l'argile.

7. Utilisation d'un catalyseur pour éliminer les constituants malodorants par destruction oxydante avec de l'ozone, le catalyseur étant formé d'un support inactif portant une couche de constituants actifs d'une épaisseur de 10 à 200 µm, les constituants actifs ayant :
(i) au moins un oxyde métallique dont le métal est choisi parmi le cuivre, le manganèse, le cobalt, le fer et le nickel; et
(ii) du dioxyde de titane et/ou de l'oxyde d'argent et/ou de l'or.

Fig. 1

air ⟶ | ozone generator | (1)

| catalyst layer | (2) ⟶ | ozone analysis meter | (3)

⟶ | stinking component analysis meter | (4)